Europäisches Patentamt

European Patent Office

Office européen des brevets

(11) Publication number: **0 145 484**
**B1**

# (12) EUROPEAN PATENT SPECIFICATION

(45) Date of publication of patent specification: **01.03.89**

(51) Int. Cl.⁴: **C 07 K 9/00**

(21) Application number: **84308610.9**

(22) Date of filing: **11.12.84**

(54) Vancomycin purification improvement and product.

(30) Priority: **12.12.83 US 560300**

(43) Date of publication of application:
**19.06.85 Bulletin 85/25**

(45) Publication of the grant of the patent:
**01.03.89 Bulletin 89/09**

(84) Designated Contracting States:
**AT BE CH DE FR GB IT LI LU NL SE**

(56) References cited:
**US-A-3 067 099**

(73) Proprietor: **ELI LILLY AND COMPANY**
**307, East McCarty Street**
**Indianapolis Indiana 46285 (US)**

(72) Inventor: **Yang, Kuo Shang**
**340 Green Hill Court, Greenwood**
**Indiana 46142 (US)**

Inventor: **Abel, Ronald Gene**
**4032 Whitaker Drive**
**Indianapolis Indiana 46254 (US)**

Inventor: **Higgins, Harvey M., Jr.**
**R.R.3, Box 337, Danville**
**Indiana 46122 (US)**

(74) Representative: **Hudson, Christopher Mark et al**
**Erl Wood Manor**
**Windlesham Surrey GU20 6PH (GB)**

Courier Press, Leamington Spa, England.

## Description

Vancomycin is a glycopeptide antibiotic substance produced by fermentation of *Nocardia orientalis* (previously designated *Streptomyces orientalis*), a microorganism isolated from soil obtained from Indonesia and India. Like most antibiotics derived from fermentation, vancoycin is a multi-factor complex of several closely related and chemically similar compounds. The preparation of the material is described in detail in U.S. Patent No. 3,067,099. Vancomycin is used commercially primarily as an antibacterial agent, although it also is effective as a ruminant feed utilization enhancer; see U.S. Patent No. 3,816,618.

The preparation of commercial vancomycin has required a multi-step process involving fermentation followed by rather extensive isolation and purification steps. The fermentation whole broth generally is filtered to remove solids and mycelia and the filtrate containing the soluble antibiotic is passed over a low cross-linked cation exchange resin which retains the antibiotic activity. The resin eluate containing vancomycin is next treated with decolorizing charcoal and the soluble vancomycin is then isolated as an insoluble copper complex. The copper complex is finally treated with hydrogen sulfide under acidic conditions to solubilize the vancomycin, which can be isolated as a free base by suitable pH adjustment. The free base is employed in formulations for oral administration to animals, and may be used directly or converted to a derivative such as the hydrochloride salt for formulations as an injectable.

The commercial production of vancomycin suffers in several respects. The use of cupric salts in the isolation process requires the subsequent use of an agent such as hydrogen sulfide to spring the copper complex and to precipitate soluble salts of copper as insoluble sulfides or the like. This has resulted in a vancomycin product that sometimes has an offensive odor and is generally somewhat colored. Also, the multi-step isolation and purification process has suffered from product loss at each stage, such that the overall yield of vancomycin free base has been on the order of only thirty percent.

The present invention provides a new salt form of vancomycin that is crystalline and that can be isolated directly from an aqueous solution of relatively crude vancomycin, for instance from fermentation broth. No salt forms of vancomycin have heretofore been obtainable in crystalline form. The new crystalline form of this invention is stable for prolonged periods, and can be employed directly in pharmaceutical preparations. The invention provides an improvement in the process for isolating vancomycin by eliminating the need to form a vancomycin copper complex, improving overall yields, and providing a product having improved pharmaceutical properties.

The new crystalline composition provided by this invention is the diphosphate salt of vancomycin. The compound can be isolated from an acidic aqueous solution of vancomycin, and generally exists as a hydrate. The degree of hydration varies depending upon the extent to which the compound is subjected to drying, but normally the salt will be isolated with from 0 to about 10 moles of water of hydration. A preferred compound of the invention is crystalline vancomycin diphosphate decahydrate. The vancomycin diphosphate provided by this invention is a highly crystalline material having good stability. When hydrated the compound of the invention exhibits the following X-ray powder diffraction properties when measured with a 114.6 mm Debye-Scherrer camera using nickel-filtered copper radiation of 1.5418Å:

| Spacing, d: | Relative Intensities, $I/I_1$: |
|---|---|
| 16.52 | 1.00 |
| 12.36 | .30 |
| 10.28 | .50 |
| 8.98 | .75 |
| 7.97 | .50 |
| 7.41 | .20 |
| 6.76 | .20 |
| 6.24 | .20 |
| 5.61 | .50 |
| 5.34 | .50 |
| 4.96 | .40 |
| 4.73 | .20 |

| Spacing, d: | Relative Intensities, $I/I_1$: |
|---|---|
| 4.44 | .50 |
| 4.26 | .50 |
| 3.98 | .40 |
| 3.75 | .40 |
| 3.55 | .50 |
| 3.35 | .30 |
| 3.15 | VW |
| 3.01 | VW |
| 2.81 | VW |
| 2.66 | VW |

Note: VW means very weak

The improvement in the process for isolating and purifying vancomycin provided by this invention is accomplished by converting vancomycin from an aqueous solution, for example a fermentation aqueous broth, to a diphosphate salt and isolating the salt by filtration. In practice, vancomycin whole broth is filtered at an alkaline pH of about 8.0 to about 10. Conventional diatomaceous filter aids such as Celite® (Johns Manville, Inc.) and the like generally are employed to facilitate removal of solid impurities and fermentation mycelia. The filtrate is then passed across an ion-exchange resin such as Iona® X-236, a low cross-linked polystyrenedivinylbenzene sodium cation resin, and the vancomycin is absorbed thereon. The vancomycin is eluted from the resin by washing the resin with water and eluting with an aqueous alkaline solution of pH 9.0 to pH 11.0. A typical solvent for elution is aqueous sodium hydroxide of pH 10.0 to 11.0. The alkaline resin eluate containing vancomycin is neutralized and the activity is re-adsorbed onto a non-functional resin, for example commerical Diaion® HP-20 resin or the like. The loaded resin is eluted with a suitable solvent, preferably an aqueous alcohol solution such as 3 to 30% (v/v) aqueous ethanol or isopropanol. If desired, the aqueous alcohol can be acidified, for example by addition of phosphoric acid, thereby generating the vancomycin diphosphate during elution of the non-functional resin.

The eluate containing the vancomycin generally is concentrated by removal of a portion of the solvent, for instance by evaporation under reduced pressure. The solution ideally is concentrated until it contains 250 to 285 mg of vancomycin per ml of solution. The concentrated eluate is acidified with a phosphate source until the pH is at or below 2.0, for instance by addition of aqueous phosphoric acid. Additional phosphate anions can be added to the solution if desired, for instance by adding an alkali metal salt of dihydrogen phosphate such as sodium or potassium dihydrogen phosphate. The amount of such added phosphate source is not critical, but generally will be from 10 to 20 percent by weight of the vancomycin present in the solution. Upon cooling the aqueous acid solution to 0 to 20°C, the vancomycin diphosphate crystalline form of this invention precipitates and can be recovered by filtration. The product can be further purified if desired by conventional means such as washing the crystals with cold water or a solvent such as an alcohol, or by recrystallization from common solvents such as water, ethanol and the like.

The preparation of the new crystalline vancomycin diphosphate provided by this invention and the operation of the process improvement is more fully illustrated by the following detailed examples.

Example 1

To a stirred suspension of 10 g of vancomycin in 15 ml of water were added 2 ml of 85% w/v aqueous phosphoric acid. All particles went into solution. The solution was cooled to 0°C for sixteen hours. The crystalline precipitate that formed was collected by filtration, washed with 20 ml of isopropanol and then with 20 ml of diethyl ether, and air dried to give 7.5 g of vancomycin diphosphate, mp 194°C (dec).

A sample of the crystalline product was dried at 120°C (weight loss was 11.55% suggesting ten moles of water of crystallization) and the sample was submitted for elemental analysis.

Analysis calculated for $C_{66}H_{75}Cl_2N_9O_{24} \cdot 2H_3PO_4$

Theory: C, 48.18; H, 4.96; N, 7.66; Cl, 4.31; O, 31.12; P, 3.77.
Found: C, 47.98; H, 5.19; N, 7.41; Cl, 4.20; O, 31.04; P, 3.77.

### Example 2

Vancomycin whole aqueous fermentation broth was made alkaline to pH 9 by addition of 6N sodium hydroxide. The aqueous alkaline mixture was filtered, and the filtrate was adjusted to pH 7 and filtered again. The filtrate was passed over an Ionac X236, sodium form resin column, which was washed with water and then eluted with pH 10 borate buffer. The activity in the neutralized eluate was adsorbed on HP-20 resin. The loaded resin was washed with water and then eluted with a solution of 0.1N phosphoric acid and 10% (v/v) aqueous isopropyl alcohol. The fractions containing vancomycin were combined (2300 ml) and concentrated under reduced pressure to a volume of 65 ml. The solution was shown by bioassay to contain 258 mg/ml of vancomycin. The solution was acidified to pH 2 by addition of 6N phosphoric acid. The acidic solution was stirred at 25°C while 2.5 g of potassium dihydrogen phosphate were added portionwise. The acidic solution was cooled to about 5°C for eighteen hours. The crystalline precipitate was collected by filtration and washed with 26 ml of cold (5°C) 2% w/v aqueous potassium dihydrogen phosphate, with 13 ml of cold (5°C) deionized water, and finally with 26 ml of cold (5°C) isopropyl alcohol. The crystalline product was dried in vacuum at 42°C for twelve hours to provide 13.5 g (76.6% yield) of vancomycin diphosphate.

### Example 3

The vancomycin diphosphate crystal form provided by this invention is a highly crystalline solid when hydrated that is stable over prolonged periods of time. The stability of bulk samples of vancomycin diphosphate was determined by preparing three lots of the compound by the method of Example 2. Samples of the three individual lots were stored at 21—28°C in polyethylene containers. Various physical parameters of each of the three lots were analyzed at zero, six, twelve and eighteen months. The results of the study are presented in the following Table:

## VANCOMYCIN DIPHOSPHATE BULK STABILITY

| Vancomycin diphosphate* | Age (Mos) | Clarity (Naphlos) | Color (465 mu) | Karl Fischer (% H$_2$O) | pH | Anti-bacterial Potency (mcg/mg) | Anti-bacterial Potency (% of initial) | (% of factor B)** |
|---|---|---|---|---|---|---|---|---|
| Lot 1 | 1 | 12.6 | .098 | 3.13 | 2.53 | 989 | 100 | 88.8 |
| | 6 | 20.0 | .115 | 2.82 | 2.39 | 939 | 94.9 | 88.2 |
| | 12 | 28.0 | .114 | 2.60 | 2.68 | 911 | 92.1 | 86.2 |
| | 18 | 38.0 | .110 | 3.39 | 2.73 | 864 | 87.4 | 85.5 |
| Lot 2 | 1 | 21.6 | .145 | 2.00 | 2.54 | 935 | 100 | 88.5 |
| | 6 | 46.0 | .168 | 1.79 | 2.39 | 948 | 101.4 | 87.9 |
| | 12 | 58.6 | .172 | 1.91 | 2.66 | 871 | 93.2 | 87.1 |
| | 18 | 52.0 | .158 | 2.09 | 2.69 | 878 | 93.9 | 84.0 |
| Lot 3 | 1 | 5.0 | .105 | 4.25 | 2.53 | 893 | 100 | 87.7 |
| | 6 | 17.0 | .114 | 3.75 | 2.39 | 926 | 103.7 | 87.8 |
| | 12 | 21.3 | .113 | 3.87 | 2.69 | 854 | 95.6 | 87.3 |
| | 18 | 30.0 | .113 | 4.21 | 2.70 | 830 | 92.9 | 85.6 |

* prepared according to Example 2

** Vancomycin is comprised of several factors, the principal factor being B.

EP 0 145 484 B1

# EP 0 145 484 B1

The data in the above table demonstrates that the vancomycin diphosphate of this invention is quite stable, retaining more than about 90% of its initial biological activity over a twelve month period.

The crystalline diphosphate provided by this invention can be employed as an intermediate in the synthesis of other vancomycin salt forms, and the compound can be formulated with conventional excipients and carriers and employed in the treatment of diseases of bacterial origin.

The crystalline diphosphate salt can be readily formulated for convenient oral or parenteral administration for therapeutic and prophylactic treatment of bacterial infections. For example, the salt can be admixed with conventional pharmaceutical carriers and excipients and used in the form of tablets, capsules, elixirs, suspensions, syrups, wafers and the like. The compositions comprising vancomycin diphosphate will contain from 0.1 to 90% by weight of the active compound, and more generally 10 to 30%. The compositions may contain common carriers and excipients such as corn starch or gelatin, lactose, sucrose, microcrystalline cellulose, kaolin, mannitol, dicalcium phosphate, sodium chloride and alginic acid. Disintegrants commonly employed in formulations of this invention include croscarmellose sodium, microcrystalline cellulose, corn starch, sodium starch glycolate and alginic acid. Tablet binders that can be included are acacia, methylcellulose, sodium carboxymethylcellulose, polyvinylpyrrolidone (Povidone), hydroxypropylmethylcellulose, sucrose, starch and ethylcellulose. Lubricants, glidants and antiadherents that can be employed include magnesium stearate or other metallic stearates, stearic acid, silicone fluid, talc, waxes, oils and colloidal silica. Flavoring agents such as peppermint, oil of wintergreen, cherry flavoring or the like can also be employed. Illustrative of formulations suitable for oral administration are the following examples:

### Example 4
Preparation of tablets containing 250 mg of vancomycin as the free base.

| Ingredient | Weight |
|---|---|
| Vancomycin Diphosphate (prepared by the method of Example 2) | 282.5 mg |
| Microcrystalline Cellulose | 101.5 mg |
| Croscarmellose sodium | 12.0 mg |
| Povidone | 12.0 mg |
| Magnesium stearate | 3.0 mg |
| Stearic acid | 4.0 mg |
| Purified water | 0.16 ml |

Vancomycin diphosphate, a portion of the microcrystalline cellulose and a portion of the croscarmellose sodium are added to a suitable container and blended until homogeneous. A solution of Povidone in water is prepared. The Povidone solution is added to the blended powders. The resulting mixture is granulated, sized if necessary and dried. Remaining microcrystalline cellulose and croscarmellose sodium are added to the dried granulation. The powders are blended. Magnesium stearate and stearic acid are added and the mixture blended. The resulting powder blend is compressed into tablets with a theoretical weight of 415 mg. Each tablet contains vancomycin diphosphate equivalent to 250 mg of vancomycin free base.

### Example 5
Preparation of capsules of vancomycin diphosphate.

| Ingredient | Weight |
|---|---|
| Vancomycin Disphosphate | 282.5 mg |
| Corn Starch Flowable Powder | 117.65 mg |
| Silicone Fluid 350 Centistokes | 2.75 mg |
| Corn Starch | 147.1 mg |

Blend vancomycin diphosphate, starch flowable powder, silicone fluid 350 centistokes and starch powder in a suitable mixer until homogeneous. Fill into appropriate size hard gelatin capsules to net fill

6

weight of 550 mg. Each capsule contains vancomycin diphosphate equivalent to 250 mg of vancomycin active base.

Example 6

Vancomycin diphosphate liquid formulation

| Ingredient | Weight |
| --- | --- |
| Vancomycin Diphosphate | 5 g |
| Red Dye | 10 mg |
| Cherry Flavor | 50 mg |
| Sodium Lauryl Sulfate | 1.5 mg |
| Sucrose | 62 g |

Add the sucrose to a suitable blender. Add a premix containing a portion of the sucrose as a powder along with the red dye, cherry flavor, and the sodium lauryl sulfate. Prior to use, 60 ml of water is added to the dry blend in a bottle. The bottle is shaken until the contents dissolve in the water. Each 5 ml will contain 250 mg of vancomycin disphosphate.

Example 7

Vancomycin diphosphate suspension (Ready-to-use)

| Ingredient | Weight |
| --- | --- |
| Vancomycin Diphosphate | 5 g |
| Butylparaben | 20 mg |
| Red Dye | 10 mg |
| Cherry Flavor | 50 mg |
| Thixin R | 400 mg |
| Sucrose Powder | 30 g |
| Fractionated Coconut Oil | q.s. to 100 ml |

Heat the fractionated coconut oil. Add while mixing the butylparaben and the thixin R. Cool to room temperature. Add the sucrose powder, red dye, cherry flavor, and the vancomycin diphosphate. Pass through a colloid mill. Each 5 ml will contain 250 mg of vancomycin diphosphate.

As pointed out above, the vancomycin diphosphate crystal form of this invention can be converted to other vancomycin derivatives, for example to other salt forms such as the hydrochloride salt. Vancomycin disphosphate can be dissolved in an aqueous acid solution and the corresponding acid addition salt recovered, or it can be reacted with an aqueous alkaline solution to provide the free base, which is subsequently reacted with a mineral acid or organic acid to give the corresponding acid addition salt. A preferred method for accomplishing such conversion employs an ion exchange resin as exemplified below.

Example 8

An ion exchange resin (IRA-45) was washed with 5% wt/v aqueous sodium hydroxide and then with 5% v/v aqueous hydrochloric acid to pH 1.2. The resin was placed in a 1.7 cm × 66 cm glass column. A solution of 81 g of vancomycin diphosphate (prepared as described in Example 2) in 812.5 ml of dionized water was passed over the ion exchange resin at a flow rate of 13 ml per minute. The eluate was collected and the resin was washed with water. The eluate and wash were combined, neutralized to pH 3.5 by addition of the free base form of IRA-45 resin in 180 ml of water. The resin was removed by filtration and the filtrate (1200 ml) was analyzed. The concentration of vancomycin hydrochloride was determined by ultraviolet analysis at 280 nm and shown to be 51.17 mg/ml. The filtrate thus contained 61.4 g (94% yield) of vancomycin hydrochloride. Further purification and lyophilization provided vancomycin hydrochloride powder.

In a further embodiment of this invention vancomycin diphosphate is employed as an antibacterial agent in the treatment of diseases of bacterial origin. The compound can be suitably formulated and administered orally or parenterally to an animal suffering from a bacterial infection. The compound can also be used prophylactically and administered to subjects suspected of developing a bacterial infection. Vancomycin diphosphate is particularly effective against strains of gram-positive micro-organisms such as streptococci, staphyloccoci, *Clostridium difficile* and the like.

In practicing the antibacterial method of this invention, vancomycin diphosphate will be suitably formulated and administered in an effective amount of about 0.5 to about 25 mg/kg. A typical daily dose for an adult human will be from about 250 mg to about 1.0 g. The exact amount of compound to be administered may of course vary depending upon the particular condition and subject being treated and related factors.

The vancomycin diphosphate of this invention can additionally be employed for improving the efficiency of feed utilization by ruminants. The compound can be formulated as an animal feedstuff or premix and administered to ruminants at the rate of 20 to 40 grams of active ingredient per ton of feed. Typical animals subject to such treatment include cattle raised for meat production, sheep and related ruminants.

**Claims for the Contracting States: BE CH DE FR GB IT LI LU NL SE**

1. A process for isolating vancomycin from aqueous solution which comprises converting the vancomycin to a crystalline diphosphate salt and separating the salt from the solution.

2. The process of Claim 1 wherein the concentration of vancomycin in aqueous solution is 250 to 285 mg/ml.

3. The process of Claim 1 wherein an alkali metal dihydrogen phosphate is added to the aqueous acid solution containing vancomycin.

4. The process of Claim 3 wherein the alkali metal dihydrogen phosphate is potassium dihydrogen phosphate.

5. The process according to Claim 1 wherein phosphoric acid is added to an aqueous solution of vancomycin until the pH is at or below 2.0.

6. Crystalline vancomycin diphosphate.

7. Crystalline vancomycin diphosphate in hydrated form having essentially the following X-ray powder diffraction pattern:

| Spacing, d: | Relative Intensities, $I/I_1$: |
|---|---|
| 16.52 | 1.00 |
| 12.36 | .30 |
| 10.28 | .50 |
| 8.98 | .75 |
| 7.97 | .50 |
| 7.41 | .20 |
| 6.76 | .20 |
| 6.24 | .20 |
| 5.61 | .50 |
| 5.34 | .50 |
| 4.96 | .40 |
| 4.73 | .20 |
| 4.44 | .50 |
| 4.26 | .50 |
| 3.98 | .40 |
| 3.75 | .40 |

| Spacing, d: | Relative Intensities, $I/I_1$: |
|---|---|
| 3.55 | .50 |
| 3.35 | .30 |
| 3.15 | VW |
| 3.01 | VW |
| 2.81 | VW |
| 2.66 | VW |

8. Crystalline vancomycin diphosphate decahydrate.

9. Crystalline vancomycin diphosphate for use as a pharmaceutical.

10. A process for preparing vancomycin hydrochloride comprising reacting vancomycin diphosphate with a hydrogen chloride source.

11. The process of Claim 10 wherein the hydrogen chloride source is an ion exchange resin wherein the available ions are proton and chloride.

**Claims for the Contracting State: AT**

1. A process for isolating vancomycin from aqueous solution which comprises converting the vancomycin to a crystalline diphosphate salt and separating the salt from the solution.

2. The process of Claim 1 wherein the concentration of vancomycin in aqueous solution is 250 to 285 mg/ml.

3. The process of Claim 1 wherein an alkali metal dihydrogen phosphate is added to the aqueous acid solution containing vancomycin.

4. The process of Claim 3 wherein the alkali metal dihydrogen phosphate is potassium dihydrogen phosphate.

5. The process according to Claim 1 wherein phosphoric acid is added to an aqueous solution of vancomycin until the pH is at or below 2.0.

6. A process for preparing vancomycin hydrochloride comprising reacting vancomycin diphosphate with a hydrogen chloride source.

7. The process of Claim 6 wherein the hydrogen chloride source is an ion exchange resin wherein the available ions are proton and chloride.

**Patentansprüche für die Vertragsstaaten: BE CH DE FR GB IT LI LU NL SE**

1. Verfahren zur Isolierung von Vancomycin aus einer wäßrigen Lösung, dadurch gekennzeichnet, daß das Vancomycin in ein kristallines Diphosphatsalz überführt wird und das Salz von der Lösung abgetrennt wird.

2. Verfahren nach Anspruch 1, dadurch gekennzeichnet, daß eine wäßrige Lösung verwendet wird, die Vancomycin in einer Konzentration von 250 bis 285 mg pro ml enthält.

3. Verfahren nach Anspruch 1, dadurch gekennzeichnet, daß die Vancomycin, enthaltende wäßrige saure Lösung mit einem Alkalimetalldihydrogenphosphat versetzt wird.

4. Verfahren nach Anspruch 3, dadurch gekennzeichnet, daß Kaliumdihydrogenphosphat als Alkalimetalldihydrogenphosphat verwendet wird.

5. Verfahren nach Anspruch 1, dadurch gekennzeichnet, daß eine wäßrige Lösung von Vancomycin so lange mit Phosphorsäure versetzt wird, bis ein pH-Wert von 2,0 oder darunter erreicht ist.

6. Kristallines Vancomycindiphosphat.

7. Kristallines Vancomycindiphosphat in hydratisierter Form, dadurch gekennzeichnet, daß es in Pulverform im wesentlichen das folgende Röntgebeugungsspektrum aufweist:

| Interplanarabstände d | Relative Intensitäten $I/I_1$ |
|---|---|
| 16,52 | 1,00 |
| 12,36 | 0,30 |
| 10,28 | 0,50 |
| 8,98 | 0,75 |

| Interplanarabstände d | Relative Intensitäten I/I₁ |
|---|---|
| 7,97 | 0,50 |
| 7,41 | 0,20 |
| 6,76 | 0,20 |
| 6,24 | 0,20 |
| 5,61 | 0,50 |
| 5,34 | 0,50 |
| 4,96 | 0,40 |
| 4,73 | 0,20 |
| 4,44 | 0,50 |
| 4,26 | 0,50 |
| 3,98 | 0,40 |
| 3,75 | 0,40 |
| 3,55 | 0,50 |
| 3,35 | 0,30 |
| 3,15 | ss |
| 3,01 | ss |
| 2,81 | ss |
| 2,66 | ss |

8. Kristallines Vancomycindiphosphatdecahydrat.

9. Kristallines Vancomycindiphosphat zur Verwendung als Pharmazeutikum.

10. Verfahren zur Herstellung von Vancomycinhydrochlorid, dadurch gekennzeichnet, daß Vancomycindiphosphat mit einer Chlorwasserstoffquelle umgesetzt wird.

11. Verfahren nach Anspruch 10, dadurch gekennzeichnet, daß die Chlorwasserstoffquelle ein Ionenaustauscherharz ist, in welchem die verfügbaren Ionen Proton und Chlorid sind.

**Patentansprüche für den Vertragsstaat: AT**

1. Verfahren zur Isolierung von Vancomycin aus einer wäßrigen Lösung, dadurch gekennzeichnet, daß das Vancomycin in ein kristallines Diphosphatsalz überführt wird und das Salz von der Lösung abgetrennt wird.

2. Verfahren nach Anspruch 1, dadurch gekennzeichnet, daß eine wäßrige Lösung verwendet wird, die Vancomycin in einer Konzentration von 250 bis 285 mg pro ml enthält.

3. Verfahren nach Anspruch 1, dadurch gekennzeichnet, daß die Vancomycin, enthaltende wäßrige saure Lösung mit einem Alkalimetalldihydrogenphosphat versetzt wird.

4. Verfahren nach Anspruch 3, dadurch gekennzeichnet, daß Kaliumdihydrogenphosphat als Alkalimetalldihydrogenphosphat verwendet wird.

5. Verfahren nach Anspruch 1, dadurch gekennzeichnet, daß eine wäßrige Lösung von Vancomycin so lange mit Phosphorsäure versetzt wird, bis ein pH-Wert von 2,0 oder darunter erreicht ist.

6. Verfahren zur Herstellung von Vancomycinhydrochlorid, dadurch gekennzeichnet, daß Vancomycindiphosphat mit iner Chlorwasserstoffquelle umgesetzt wird.

7. Verfahren nach Anspruch 6, dadurch gekennzeichnet, daß die Chlorwasserstoffquelle ein Ionenaustauscherharz ist, in welchem die verfügbaren Ionen Proton und Chlorid sind.

**Revendications pour les Etats contractants: BE CH DE FR GB IT LI LU NL SE**

1. Procédé en vue d'isoler la vancomycine d'une solution aqueuse, caractérisé en ce qu'il consiste à

10

EP 0 145 484 B1

convertir la vancomycine en un sel diphosphate cristallin et séparer le sel de la solution.

2. Procédé selon la revendication 1, caractérisé en ce que la concentration en vancomycine dans la solution aqueuse est de 250 à 285 mg/ml.

3. Procédé selon la revendication 1, caractérisé en ce qu'on ajoute un dihydrogénophosphate d'un métal alcalin à la solution acide aqueuse contenant de la vancomycine.

4. Procédé selon la revendication 3, caractérisé en ce que le dihydrogénophosphate d'une métal alcalin est le dihydrogénophosphate de potassium.

5. Procédé selon la revendication 1, caractérisé en ce qu'on ajoute de l'acide phosphorique à une solution aqueuse de vancomycine jusqu'à ce que le pH soit égal ou inférieur à 2,0.

6. Diphosphate de vancomycine cristallin.

7. Diphosphate de vancomycine cristallin sous forme hydratée ayant essentiellement le diagramme de diffraction de poudre aux rayons X ci-après:

| Espacement d | Intensités relatives, $I/I_1$ |
|---|---|
| 16,52 | 1,00 |
| 12,36 | 0,30 |
| 10,28 | 0,50 |
| 8,98 | 0,75 |
| 7,97 | 0,50 |
| 7,41 | 0,20 |
| 6,76 | 0,20 |
| 6,24 | 0,20 |
| 5,61 | 0,50 |
| 5,34 | 0,50 |
| 4,96 | 0,40 |
| 4,73 | 0,20 |
| 4,44 | 0,50 |
| 4,26 | 0,50 |
| 3,98 | 0,40 |
| 3,75 | 0,40 |
| 3,55 | 0,50 |
| 3,35 | 0,30 |
| 3,15 | très faible |
| 3,01 | très faible |
| 2,81 | très faible |
| 2,66 | très faible |

8. Diphosphate de vancomycine cristallin à dix molécules d'eau.

9. Diphosphate de vancomycine cristallin destiné à être utilisé comme produit pharmaceutique.

10. Procédé de préparation de chlorhydrate de vancomycine, caractérisé en ce qu'il consiste à faire réagir du diphosphate de vancomycine avec une source de chlorure d'hydrogène.

11. Procédé selon la revendication 10, caractérisé en ce que la source de chlorure d'hydrogène est une résine échangeuse d'ions dans laquelle les ions disponibles sont un proton et un chlorure.

11

# EP 0 145 484 B1

**Revendications pour l'Etat contractant: AT**

1. Procédé en vue d'isoler la vancomycine d'une solution aqueuse, caractérisé en ce qu'il consiste à convertir la vancomycine en un sel diphosphate cristallin et séparer le sel de la solution.

2. Procédé selon la revendication 1, caractérisé en ce que la concentration en vancomycine dans la solution aqueuse est de 250 à 285 mg/ml.

3. Procédé selon la revendication 1, caractérisé en ce qu'on ajoute un dihydrogénophosphate d'un métal alcalin à la solution aqueuse acide contenant de la vancomycine.

4. Procédé selon la revendication 3, caractérisé en ce que le dihydrogénophosphate d'une métal alcalin est le dihydrogénophosphate de potassium.

5. Procédé selon la revendication 1, caractérisé en ce que l'acide phosphorique est ajoute à une solution aqueuse de vancomycine jusqu'à ce que le pH soit égal ou inférieur à 2,0.

6. Procédé de préparation de chlorhydrate de vancomycine, caractérisé en ce qu'il consiste à faire réagir du diphosphate de vancomycine avec une source de chlorure d'hydrogène.

7. Procédé selon la revendication 6, caractérisé en ce que la source de chlorure d'hydrogène est une résine échangeuse d'ions dans laquelle les ions disponibles sont un proton et un chlorure.